# EUROPÄISCHE PATENTANMELDUNG

(11) **EP 2 201 951 A1**
(43) Veröffentlichungstag der Anmeldung: **30.06.2010**
(21) Anmeldenummer: 08450183.2
(22) Anmeldetag: 14.11.2008
(51) Int. Cl.: A61K 31/44, A61K 47/26, A61K 47/34, A01N 43/40, A61K 8/34, A61K 8/49, A61K 8/60

(54) **Octenidin-Zusammensetzung**

(71) Anmelder: Aydinoglu, Ahmet Melih, Balgat, Ankara (TR)
(72) Erfinder: Aydinoglu, Ahmet Melih, Balgat, Ankara (TR)
(74) Vertreter: Sonn & Partner Patentanwälte

(57) **Zusammenfassung**

Die Erfindung betrifft Octenidin oder ein pharmazeutisches Salz hiervon, insbesondere Octenidindihydrochlorid, bestimmt zur gemeinsamen therapeutischen Verabreichung des Octenidins in Lösung mit einem Polyalkohol der Formel 1: (H-C-OH)ₐ(HO-C-OH)_{b}(H-C-H)_{c},
wobei a, b, c ganze Zahlen sind, wobei a+b mindestens 2, vorzugsweise mindestens 3 ist, c ausgewählt ist aus 0, 1 oder einer Zahl aus dem Bereich von 2 bis a+b,
gegebenenfalls zusätzlich mit einer oder mehrerer Aldehydgruppen sofern diese (zyklische) Acetale mit einer der Hydroxygruppen bilden oder ein oder mehreren Ketogruppen gegebenenfalls als Acetal mit einer der Hydroxygruppen,
gegebenenfalls zusätzlich mit einer oder mehrerer Carbonsäuregruppen sofern der Polyalkohol ein zyklisches Acetal oder Acetal ist, vorzugsweise mit Ringgrößen von 5 bis 7 Atomen, bzw. eines Polymers, Polyethers oder Polyesters hiervon, mit der Maßgabe, dass sofern a+b 2 oder 3 ist, der Polyalkohol als Polymer, Polyether oder Polyester vorliegt mit mindestens zwei Einheiten der Formel 1 sowie Kits enthaltend diese Wirkstoffe, und deren Verwendung zur Infektions- und Wundbehandlung.

## Beschreibung

Die vorliegende Erfindung betrifft das Gebiet desinfizierender und antiseptischer Formulierungen sowie deren Anwendungen.

Octenidindihydrochlorid ist ein mikrobiozider Wirkstoff, der vor allem in Haut-, Schleimhaut- und Wundantiseptika eingesetzt wird. Die Vermarktung erfolgt unter dem Handelsnamen Octenisept^{®}. Auf Grund seines breiten Wirkungsspektrums auf der einen Seite und der guten Verträglichkeit auf der anderen Seite gewinnt dieser Wirkstoff zunehmend an Bedeutung in der Praxis der Antiseptik und hat in einigen Indikationsbereichen klassische antiseptische Wirkstoffe wie Chlorhexidin, Triclosan und PVP-Iod bereits abgelöst.

Octenidin wird stark an negative Zelloberflächen adsorbiert. Dort reagiert es mit anionischen Polysacchariden der mikrobiellen Zellwand und Phospholipiden der Zellmembran, greift in die enzymatischen Systeme ein, stört Zellfunktionen und führt zur Leckage der Plasmamembran. Dadurch wird die Funktion der Mitochondrien gestört. Untersuchungen zeigen eine starke Adhärenz an Lipidkomponenten in Zellmembranen (z.B. Cardiolipin), was die hohe antimikrobielle Wirkung bei gleichzeitig guter Verträglichkeit für menschliches Epithel und Wundgewebe erklärt. Bei 30 min Einwirkungszeit ist Octenidin in der Verdünnung von 22 bzw. 17 mg/l (= 0,0022% bzw. 0,0017%) gegenüber E. coli bzw. S. aureus wirksam (Kramer A, Müller G; Octenidindihydrochlorid; in: Wallhäußers Praxis der Sterilisation, Desinfektion, Antiseptik und Konservierung ; Stuttgart: Thieme; 2007).

Octenidin bzw. Octenidindihydrochlorid und dessen Derivate werden in den US-Patenten US 4,206,215 und US 4,442,125 als anti-mikrobielle Substanz beschrieben.

Die DE 3 925 540 C1 betrifft eine wässrige antiseptische Zusammensetzung, umfassend Octenidin sowie Phenoxyethanol und/oder Phenoxypropanol, welche als Lösungsvermittler eingesetzt werden.

Die DE 10 2004 052 308 A1 und WO 2006/039961 betreffen Lutschtabletten, umfassend Octenidin, welche in einer festen Matrix aus Zucker vorgesehen wird. Diese Matrix ist eine feste Masse aus Zucker im Bereich von 70-99,95 Gew.-%, welche als Träger dient und den bitteren Geschmack von Octenidin überdeckt.

Die WO 2007/023066 A1 betrifft eine Lösung aus Octenidindihydrochlorid und einen ein- oder zweiwertigen Alkohol bzw. Glycerin. Diese Alkohole werden als Alternative zum herkömmlichen Phenoxyethanol oder Phenoxypropanol früherer Octenidindihydrochlorid-Präparationen angesehen.

Die WO 2007/031519 A2 betrifft Octenidindihydrochlorid-Präparationen in Form von einhüllenden Liposomen. Durch die Verwendung von Phospholipidliposomen soll die Zytotoxizität von Octenidin gesenkt werden.

Trotz der Vorteile von Octenidin wäre es wünschenswert ein Präparat zu Erhalten welches verbesserte Wirkungen insbesondere bei der therapeutischen Anwendung zur Wund- und Schleimhautantiseptik aufweist.

Daher betrifft die vorliegende Erfindung in einem Aspekt Octenidin oder ein pharmazeutisches Salz hiervon, insbesondere Octenidindihydrochlorid, bestimmt zur gemeinsamen therapeutischen Verabreichung des Octenidins in Lösung mit einem Polyalkohol der Formel 1: (H-C-OH)ₐ(HO-C-OH)_{b}(H-C-H)_{c},
wobei a, b, c ganze Zahlen sind, wobei a+b mindestens 2, vorzugsweise mindestens 3 ist, c ausgewählt ist aus 0, 1 oder einer Zahl aus dem Bereich von 2 bis a+b,
gegebenenfalls zusätzlich mit einer oder mehrerer Aldehydgruppen sofern diese (zyklische) Halbacetale oder Acetale mit einer der Hydroxygruppen bilden und/oder ein oder mehreren Ketogruppen gegebenenfalls als Acetal (bzw. Ketal) mit einer der Hydroxygruppen,
gegebenenfalls zusätzlich mit einer oder mehrerer Carbonsäuregruppen sofern der Polyalkohol ein zyklisches Halbacetal oder Acetal ist, vorzugsweise mit Ringgrößen von 5 bis 7 Atomen, bzw. eines Polymers, Polyethers oder Polyesters hiervon, mit der Maßgabe, dass sofern a+b 2 oder 3 ist der Polyalkohol als Polymer, Polyether oder Polyester vorliegt mit mindestens zwei Einheiten der Formel 1. In einem besonders bevorzugten Aspekt betrifft die vorliegende Erfindung eine pharmazeutische Zusammensetzung mit Octenidin und Glukose als Gemisch oder als getrennte Inhaltsstoffe in einem Kit zur gemeinsamen Verabreichung.

Vorzugsweise betrifft die vorliegende Erfindung in einem Aspekt eine pharmazeutische Zusammensetzung umfassend eine Lösung von Octenidin, vorzugsweise Octenidindihydrochlorid, oder eines seiner pharmazeutisch akzeptablen Salze und Glukose mit einer Glukosekonzentration von 0,01% bis 12% (w/v), vorzugsweise von 0,1% bis 10%, insbesondere bevorzugst von 0,5% bis 7,5%, am meisten bevorzugt von 2,5% bis 5% und einer Octenidinkonzentration von 0,0001% bis 5% (w/w), vorzugsweise von 0,001% bis 1%, besonders bevorzugt von 0,002% bis 0,5%, gegebenenfalls zusammen mit einem Träger. Besondere und weitere Erfindungsgegenstände werden in den Ansprüchen definiert.

Octenidin ist ein hochwirksames bakteriostatisches/bakterizides Agens das vielfach in der Wundreinigung und präoperativ zur Desinfektion des Operationsareals eingesetzt wird, insbesondere als Dihydrochlorid Salz. Es wird bisher meist als eine wässrige Lösung in der Konzentration 0,1% (w/v) oder als eine Verdünnung 1 : 1 mit NaCl_{physiol} angewendet. Die ausgezeichnete Hemmung auf Prokaryonten birgt aber auch gewisse Nachteile für das Wachstum eukaryontischer Zellen, die bei korrekter Wundheilung proliferieren und differenzieren müssen, um einen gut haftenden, dichten Wundverschluss herzustellen. Die Stabilität der zellulären Abdeckung ist von der Ausbildung molekularer Interaktion der Gewebematrix mit Zelloberflächenmolekülen abhängig.

Erfindungsgemäß wurde gezeigt, dass die Verwendung von NaCl_{physiol} eine Präzipitation bei Octenisept^{®} bewirkt, die die Wirksamkeit mindert. Es konnte weiters gezeigt werden, dass dieser Nachteil durch Zusatz von Polyalkoholen, insbesondere Glukose vermieden werden kann. In bestimmten Konzentrationen angewendet begünstigt diese Formulierung die verstärkte Ausbildung von Zell-Matrixinteraktion, in der Folge Zelladhäsion genannt, welche in kultivierten fibroblastoiden Zellen mittels Zelladhäsions-Assay gemessen wurde. Mit diesem Messverfahren wurde die Wirkungsweise der erfindungsgemäßen Zusammensetzung gemessen und die optimale Polyalkohol-, insbesondere Glukosekonzentration belegt.

Die Interaktion von Octenidin und Bakterien ist ein Faktor für seine Wirksamkeit. Nach bisherigen Erkenntnissen scheint die negativ geladene Bakterienoberfläche zur Bindung von positiv geladenem Octenidin wesentlich verantwortlich zu sein. Appliziert man wie vielfach üblich Octenisept^{®} in einer 1:1 Verdünnung mit physiologischer Kochsalzlösung, so wird angenommen - ohne auf eine bestimmte Theorie beschränkt zu sein -, dass die sich um das Octenidin-Molekül bildende Mischhydrathülle aus NaCl und dem Dipol Wasser zu einer Reduktion der wirksamen positiven Ladung und in der Folge zur Minderung der Interaktion mit der Bakterienwand führt. Erfindungsgemäß wurde beobachtet, dass der Zusatz von Polyalkoholen, insbesondere Glukose, die Wundheilung verbesserte ohne die antiseptische Wirkung zu beeinträchtigten bzw. konnte diese sogar gesteigert werden.

In den Beispielen wurde gezeigt, dass die Anwendung einer Verdünnung mit NaCl zur Bildung eines Niederschlags führt. In diesem Niederschlag wird Octenidin als inaktiver, die Verfügbarkeit von bakterizider Wirkung maskierender, kristalliner Fremdkörper gefunden und wird da er in Wunden überdies mechanische Reizung ausübt unerwünscht in der Wundheilung interagieren. Diese Interaktion kann durch Verdünnung mit ungeladenen Kohlenhydraten wie z.B. mit 5% Glukose-Lösung verhindert werden.

Octenidin wirkt vordergründig durch seine Affinität an Bakterienoberflächen antibakteriell. Zusätzlich weist Octendidin eine desinfizierende/antimikrobielle Wirkung gegen Pilze und Viren auf, welche sich durch Verabreichung mit einem Polyalkohol gemäß der Erfindung voll entfalten kann. Der Polyalkohol unterstützt zudem eine für die Wundheilung erwünschte Stabilisierung des Zellverbandes, wie aus dem in den Beispielen gezeigten Zelladhäsionstest ersichtlich ist.

Die erfindungsgemäßen Zusammensetzungen zeigen einerseits die Wirksamkeit in Bezug auf die Verbesserung der Zelladhäsion, die experimentell im Bereich von 1 g/l bis 25 g/l (0,1% bis 2,5%) als besonders bevorzugt belegt wurde. Andererseits liegt bei der Verwendung von hypotonen Octenisept aufgrund der Wirkungsweise der Rezeptoren und Ionenkanäle die in der Nociception involviert sind ein verstärktes Auftreten von Wundschmerz vor. Es wird durch die Anwendung von den erfindungsgemäßen Zusammensetzungen zur vorgeschlagenen gemeinsamen Verabreichung an Wunden und sensiblen Schleimhäuten mit ungefähr isotonen Lösungen (entsprechend ∼5% Glukose) oder fallweise auch etwas hypotonen Lösungen (entsprechend 2,5% Glukose) die negative Wirkung auf die Nociception reduziert bzw. eliminiert. Damit wird Schmerzfreiheit und eine substantielle Verbesserung der Behandlung erreicht und insbesondere auch eine Folgereaktion bei Anwendungen wie am Peritoneum eine Flüssigkeitsverschiebung vermieden.

Gemäß der vorliegenden Erfindung wird somit Octenidin zusammen mit einem Polyalkohol zur gemeinsamen Verabreichung vorgesehen. Dieser Polyalkohol umfasst vorzugsweise eine Grundstruktur der Formel 1: (H-C-OH)ₐ(HO-C-OH)_{b}(H-C-H)_{c}. Vorzugsweise sind a+b dabei mindestens 2, mindestens 3, mindestens 4, mindestens 5, mindestens 6 oder auch mindestens 7. Für sich genommen ist a vorzugsweise 0, 1, 2, 3, 4, 5, 6 oder auch mindestens 7. In Kombination bzw. unabhängig hiervon kann b ausgewählt sein aus 0, 1, 2, 3, 4, 5, 6 oder mindestens 7. Die Definitionen von a und b legen die Anzahl der erfindungsgemäß bedeutsamen Hydroxylgruppen fest. Insbesondere ist bevorzugt, dass die Polyalkohole mindestens 4, vorzugsweise mindestens 5, 6, 7 oder auch mindestens 8 Hydroxylgruppen aufweisen. Im Fall von a+b = 2 oder 3, in weiteren speziellen Fällen auch mit a+b = 4 oder 5, wird daher vorzugsweise vorgesehen, dass die Grundstruktur der obigen Formel 1 als Polymer, insbesondere Polykondensat, Polyether oder Polyester vorliegt. Selbstverständlich können auch größere Strukturelemente der Formel 1 als solche Polymere, Polykondensate, Polyether oder Polyester vorliegen, wobei ein verknüpfendes O bzw. OC(O) der Polyether oder Polyester nicht in obiger Formel angegeben wurde und als zusätzliches verknüpfendes Strukturelement möglich ist. Die Bezeichnung von "Poly" soll hierbei als Angabe der obigen Strukturelemente, die mindestens 2-fach, vorzugsweise 3-fach, insbesondere bevorzugt 4-fach, speziell bevorzugt 5-fach oder auch mindestens 6-fach vorkommen können, verstanden werden. Im Falle von Polymeren, vorzugsweise Polyglycolen, können bis zu 5000, bis zu 4000, bis zu 3000, bis zu 2000, bis zu 1000, bis zu 800, bis zu 500 oder bis zu 300 Elemente der Formel 1 vorgesehen werden.

Die Elemente können dabei einheitlich bzw. homogen aus demselben Grundelement aufgebaut sein, bzw. heterogen aus Mischungen hiervon. Homogen sind vorzugsweise Polyglykole, heterogen vorzugsweise Kohlenhydratpolymere aus Zuckermonomeren bzw. Derivate hiervon.

Die erfindungsgemäßen Polyalkohole können gegebenenfalls zusätzlich mit einer oder mehreren Aldehydgruppen vorgesehen werden, sofern diese (zyklische) Halbacetale oder Acetale mit einer der Hydroxygruppen bilden oder ein oder mehrere Ketogruppen, gegebenenfalls als Acetal mit einer Hydroxygruppe vorgesehen werden. Die Ausbildung von Aldehyden als Acetale ist insbesondere bevorzugt, da Aldehydgruppen potenziell toxisch aufgrund ihrer hohen Reaktivität wirken. Im Speziellen liegt daher das Aldehyd im Gleichgewicht mit seinem Acetal, vorzugsweise zu mindestens 95%, insbesondere mindestens 98%, speziell bevorzugt mindestens 99%, als Acetal im Gleichgewicht zur offenen Aldehydstruktur bei physiologischen Bedingungen, insbesondere bei 37°C, vor. Sofern eine Ketogruppe vorgesehen ist, liegt diese in ebenfalls bevorzugten Ausführungsformen zumindest zu 95%, vorzugsweise mindestens 98%, mindestens 99% als Acetal mit einer der Hydroxygruppen des Polyalkohols (intramolekular) im Gleichgewicht bei physiologischen Bedingungen, insbesondere 37°C, vor. Die Halbacetale bzw. Acetale bilden vorzugsweise intramolekulare Ringe mit 5, 6, 7, 8 oder auch 9 Atomen. Vorzugsweise umfasst der Polyalkohol, insbesondere in Form eines Acetals, ein Glykosid.

Gegebenenfalls kann der Polyalkohol der Grundstruktur der Formel 1 eine Karbonsäuregruppe aufweisen, sofern der Polyalkohol ein zyklisches Halbacetal oder Acetal ist, vorzugsweise mit Ringgrößen von 5-7 Atomen. Obwohl erfindungsgemäß festgestellt wurde, dass Säuregruppen weniger wirksam zusammen mit Octenidin sind, wurde beispielsweise anhand der Glukuronsäure gezeigt, dass dennoch eine erhöhte Wirksamkeit (im Vergleich zu herkömmlichen Octenisept^{®}) zu verzeichnen ist. Glukuronsäure ist eine Karbonsäure, welche analog zu Glukose als 6-atomisches zyklisches Acetal vorliegt, wobei das sechste Kohlenstoffatom zur Karbonsäure oxidiert ist.

Ebenso zu dem zweckgebundenen Octenidin bzw. des pharmazeutischen Salzes hiervon betrifft die vorliegende Erfindung in einem weiteren Aspekt einen Kit umfassend einen Polyalkohol wie oben definiert und Octenidin bzw. pharmazeutische Salze hiervon, insbesondere Octenidindihydrochlorid, vorzugsweise bestimmt zur gemeinsamen therapeutischen Verabreichung in Lösung. Insbesondere Octenidin bzw. dessen Salz kann gemäß des Kits als gelöstes Octenidin vorgesehen werden. Alternativ bzw. zusätzlich kann der Polyalkohol bereits gelöst sein. Eine solche Lösung ermöglicht eine rasche Anwendung von Octenidin, insbesondere zur Desinfektion.

In einem weiteren Aspekt betrifft die vorliegende Erfindung eine pharmazeutische Zusammensetzung in Form einer Lösung, umfassend einen Polyalkohol wie oben definiert und Octenidin oder pharmazeutische Salze hiervon, insbesondere Octenidindihydrochlorid. Ebenfalls offenbart werden Mischungen des Polyalkohols mit Octenidin als pharmazeutische Zusammensetzung. Feste Mischungen können insbesondere zur späteren Auflösung unmittelbar vor der Verabreichung vorgesehen werden.

Vorzugsweise ist der Polyalkohol gemäß der vorliegenden Erfindung ein Kohlenhydrat der Summenformel 2: Cₙ(H₂O)ₘ, wobei n eine ganze Zahl von mindestens 3, vorzugsweise 4, 5, 6, 7, 8, 9, 10, 11 oder mindestens 12 ist und m eine ganze Zahl im Bereich von n-15% bis n+15% (auf ganze Zahlen gerundet), vorzugsweise n-1, n oder n+1. Die Rundung erfolgt hierbei mathematisch, d.h. Kommastellen von ..,5 werden aufgerundet bzw...,4999... abgerundet. Selbstredend können auch diese Kohlenhydrate polymerisiert werden, insbesondere polykondensiert, zu löslichen Kohlenhydratoligo- oder polymeren. Insbesondere die Fähigkeit zur Bildung von Lösungen ohne Trübungen durch den Polyalkohol ist besonders bevorzugt. Obwohl in besonders bevorzugten Ausführungsformen die Präparation gemäß vorliegender Erfindung als Gel, insbesondere Hydrogel, vorliegt, wird der erfindungsgemäße Polyalkohol nicht als Gelbildner verstanden, insbesondere da ein Gelbildner nicht als lösliche Substanz angesehen wird sondern als solvatisierte semi-feste Struktur.

In besonders bevorzugten Ausführungen ist der Polyalkohol ungeladen. Obwohl auch schwach saure Polyalkohole, wie beispielsweise die Glukuronsäure, möglich sind, werden die besten Ergebnisse mit ungeladenen Polyalkoholen erzielt. Sofern der Polyalkohol eine schwache Säure ist, ist vorzugsweise der pKa-Wert des Polyalkohols größer als 3, besonders bevorzugt größer als 3,5, größer als 4, größer als 4,5 oder größer als 5.

In besonders bevorzugten Ausführungsformen ist der Polyalkohol ein Mono- oder Disaccharid bzw. ein Dioxi- oder Monokarbonsäurederivat eines Mono- oder Disaccharids. In weiteren speziellen Ausführungsformen umfasst der Polyalkohol eine Aldose oder Ketose, vorzugsweise eine Aldohexose, Aldopentose, Ketohexose oder eine Ketopentose. Besonders bevorzugte Beispiele der erfindungsgemäßen Polyalkohole sind Glukose, Galactose, Fruktose, Fukose, Maltose, Ripose, Dioxiripose, Dioxiglukose, insbesondere 2-Dioxiglukose, Saccharose, Lactose, Glukuronsäure, Sorbitol, insbesondere Disorbitol, Polyethylenglycol, Dextrose, Isomalt, Maltitsirup, Polyglukose, Glukane und Fruktooligosaccharide.

Vorzugsweise werden Octenidin bzw. der Polyalkohol in wässrigen Lösungen, vorzugsweise reinem Wasser gelöst. Gegebenenfalls können geringe Alkoholanteile als Lösungsmittel vorgesehen werden, beispielsweise Ethanol, Propanol oder Isobutanol. In solchen Fällen ist vorzugsweise der Alkoholgehalt unter 5%, unter 3%, unter 2% oder unter 1%.

Vorzugsweise wird der Polyalkohol in Lösung zur Verabreichung mit Octenidin bei einer Konzentration von mindestens 0,001%, mindestens 0,005% oder mindestens 0,01%, vorzugsweise von mindestens 0,025%, insbesondere bevorzugt von mindestens 0,1%, mindestens 0,5%, mindestens 1% am meisten bevorzugt von mindestens 2,5% (w/v), vorgesehen. Die Konzentrationsangabe (w/v) betrifft hierin eine Konzentration von Gewicht pro Volumen. Vorzugsweise ist die Maximalkonzentration des Polyalkohols in Lösung zur Verabreichung mit Octenidin bei 12%, vorzugsweise 10%, insbesondere bis 7,5%, am meisten bevorzugt bis 5% (w/v).

In weiteren besonders bevorzugten Ausführungsformen hat Octenidin in Lösungen zur Verabreichung mit dem Polyalkohol bzw. in der gemeinsamen Lösung der Zusammensetzung oder im Kit eine Octenidinkonzentration von mindestens 0,0001%, insbesondere bevorzugt mindestens 0,001%, besonders bevorzugt mindestens 0,002%, mindestens 0,005%, mindestens 0,01% oder mindestens 0,05% (w/w). In weiteren besonders bevorzugten Ausführungsformen ist die Octenidinkonzentration bis 5%, bis 1%, bis 0,5% oder bis 0,2% (w/w).

Obwohl erfindungsgemäß festgestellt wurde, dass der Polyalkohol gemäß der vorliegenden Erfindung eine maskierende Wirkung von Salz, insbesondere NaCl auf Octenidin verhindern kann, ist bevorzugt, dass die Salzkonzentration, insbesondere NaCl-Konzentration maximal 0,5% (w/v), vorzugsweise maximal 0,1%, maximal 0,05%, maximal 0,01%, besonders bevorzugt maximal 0,005%, am meisten bevorzugt maximal 0,001%, ist.

In speziellen Ausführungsformen liegt der Polyalkohol und/oder Octenidin in der Präparation von zweckgebundenem Octenidin des Kits oder der Zusammensetzung in einem Träger vor. Der Träger ist vorzugsweise ausgewählt aus einem Gel, vorzugsweise einem Hydrogel oder einem Wundverband oder Tupfer, der gegebenenfalls mit einer Lösung enthaltend dem Polyalkohol und/oder Octenidin getränkt ist. Weitere Träger umfassen langsam-freisetzende Träger, welche die Wirkstoffkombination als länger wirksame Applikation zeitverzögert bzw. verlangsamt freisetzt. Eine solche Präparation mit einem entsprechenden Träger ist besonders zur topischen und raschen Anwendung geeignet.

Das Octenidin, der Kit oder die Zusammensetzung gemäß der vorliegenden Erfindung mit Octenidin und dem Polyalkohol zur gemeinsamen therapeutischen Verabreichung wird insbesondere zur Behandlung von Wunden oder Verbrennungen, insbesondere der Haut, einer Schleimhaut, insbesondere der Vagina, der Bauchhöhle, von inneren Organen, insbesondere bei chirurgischen Eingriffen oder als Prophylaxe vor chirurgischen Eingriffen oder zur Behandlung und Vorbeugung von Infektionen, insbesondere Wundinfektionen, vorzugsweise Infektionen von Verbrennungswunden, insbesondere der Haut, einer Schleimhaut, insbesondere der Vagina, der Bauchhöhle, von inneren Organen, insbesondere bei chirurgischen Eingriffen oder als Prophylaxe vor chirurgischen Eingriffen,bestimmt. In einem weiteren Aspekt betrifft die vorliegende Erfindung den Polyalkohol, vorzugsweise Glukose, bestimmt zur Erhöhung der Wirkung von Octenidin bei seiner therapeutischen Verwendung; sowie Octenidin bzw. eines pharmazeutischen Salzes hiervon, wie z.B. Octenidindihydrochlorid, bestimmt zur gemeinsamen therapeutischen Verabreichung mit dem Polyalkohol, vorzugsweise Glukose.

Die Anwendung von Glukose 5% als leicht handhabbares, überall erhältliches und leicht berechenbares Diluens erlaubt Octenidin mit ungehemmter Affinität an die Bakterienoberflächen zu binden, da die bakteriostatische Wirkung voll vorhanden ist und unterstützt andererseits eine für die Wundheilung erwünschte Stabilisierung des Zellverbandes, wie aus den Zelladhäsiontests ersichtlich ist.

In einem weiteren Aspekt betrifft die vorliegende Erfindung einen Kit umfassend Glukose und Octenidin oder ein pharmazeutisches Salz hiervon, insbesondere Octenidindihydrochlorid. Der erfindungsgemäße Effekt der verbesserten Wundheilung tritt insbesondere bei der gemeinsamen Verabreichung dieses Kits ein. Vorgesehen werden kann auch ein Kit, in dem Glukose und Octenidin getrennt vorgesehen werden. Die Inhaltsstoffe können insbesondere zur gemeinsamen Verabreichung bestimmt sein. Vorzugsweise ist entweder Glukose oder Octenidin bzw. beide Komponenten in Lösung. Die Verwendung von gelöster Glukose bzw. Octenidin, insbesondere bevorzugt sowohl gelöste Glukose als auch gelöstes Octenidin, ermöglicht eine rasche Anwendung z.B. durch Mischen der beiden Stoffe bzw. der beiden Lösungen und nachfolgender Applikation.

Die Lösung, in der der Kit bzw. die pharmazeutische Zusammensetzung gemäß der vorliegenden Erfindung vorgesehen wird, ist vorzugsweise eine wässrige Lösung. Insbesondere kann Wasser als reines Lösungsmittel, aber auch mit geringen anderen organischen Lösungsmitteln wie beispielsweise Alkoholen, vorgesehen werden. Vorzugsweise ist der Alkoholgehalt einer solchen Lösung unterhalb 10%, insbesondere unterhalb 5%, besonders bevorzugt maximal 2% bzw. unterhalb von 1%.

Die Polyalkoholkonzentration, vorzugsweise Glukosekonzentration, in einer Polyalkohollösung des Kits zur Verabreichung mit Octenidin bzw. in der pharmazeutischen Zusammensetzung, in der beide Stoffe bereits gemischt sind, ist vorzugsweise mindestens 0,001%, mindestens 0,005% oder mindestens 0,01%, insbesondere bevorzugt mindestens 0,05% oder mindestens 0,1%, speziell bevorzugt mindestens 0,5%, mindestens 1% oder mindestens 2,5% (alle Prozentangaben w/v). Vorzugsweise ist die Polyalkoholkonzentration, vorzugsweise Glukosekonzentration, maximal 30%, maximal 20%, maximal 10%, maximal 8%, maximal 5% (w/v). Im Kit umfassend eine Polyalkohollösung und eine Octenidinlösung vorgesehen zur Mischung, z.B. im Verhältnis 1:1, sind die bevorzugten Konzentrationen entsprechend erhöht, z.B. bei einem Mischverhältnis von 1:1 doppelt, z.B. für die Polyalkohollösung wird eine Polyalkoholkonzentration von 5-10% und für die Octenidinlösung die Octenidinkonzentration von 0,004-1% am meisten bevorzugt.

Die hierin verwendete Konzentrationsangabe (w/v) bezieht sich auf Gewicht pro Volumen vom englischen weight per volume. Die ebenfalls hierin verwendete Konzentrationsangabe (w/w) betrifft Gewicht pro Gesamtgewicht der jeweiligen Präparation, Zubereitung oder Lösung.

Vorzugsweise hat die Octenidinlösung zur gemeinsamen Verabreichung mit Glukose bzw. die pharmazeutische Zusammensetzung umfassend eine Lösung von Octenidin, eine Octenidinkonzentration von mindestens 0,0001% (w/w), vorzugsweise von mindestens 0,001%, besonders bevorzugt mindestens 0,002%, mindestens 0,005%, mindestens 0,01% oder mindestens 0,05%. Vorzugsweise Maximalkonzentrationen von Octenidin sind bis zu 10% (w/w), vorzugsweise bis 5%, bis 1%, insbesondere bevorzugt bis 0,5%, im Speziellen bevorzugt bis 0,2%.

Wie hierin bereits erwähnt und auch in den Beispielen gezeigt kann ein Salzanteil, insbesondere eine hohe NaCl-Konzentration z.B. bei pysiologischen Bedingungen die Wirkung von Octenidin hemmen. Obwohl Glukose diesen Hemmeffekt verhindern kann, wird in besonderen Ausführungsformen der vorliegenden Erfindung die NaCl-Konzentration limitiert. Vorzugsweise umfasst die Octenidinlösung zur Verabreichung mit Glukose bzw. die erfindungsgemäße pharmazeutische Zusammensetzung eine Salzkonzentration, insbesondere NaCl-Konzentration von unter 0,1% (w/v), vorzugsweise unter 0,05% oder unter 0,01%, besonders bevorzugt unter 0,05%, am meisten bevorzugt unter 0,001%.

Gemäß dem Kit der vorliegenden Erfindung sowie der pharmazeutischen Zusammensetzung mit Octenidin und Glukose kann Glukose und/oder Octenidin in einem Träger vorgesehen werden. Vorzugsweise ist der Träger ein Gel, insbesondere ein Hydrogel. Ein weiterer möglicher Träger ist ein Wundverband oder Tupfer, der gegebenenfalls mit einer Lösung enthaltend Glukose und/oder Octenidin getränkt ist. Ein solcher Wundverband oder ein solches Gel kann direkt bzw. durch Zusatz der jeweils anderen Komponente ausgewählt aus Glukose und/oder Octenidin verabreicht werden. Weitere Träger umfassen langsam-freisetzende Träger, wie Mikroverkapselungen, welche die Wirkstoffkombination als länger wirksame Applikation zeitverzögert bzw. verlangsamt freisetzt. Ein Gel oder Hydrogel kann mit herkömmlichen Gel-Bildnern hergestellt werden, beispielsweise Zellulosederivate wie Hydroxyethylzellulose. Weitere mögliche Trägermedien umfassen Emulsionen, vorzugsweise Öl in Wasser oder Wasser in Öl Emulsionen und Liposomen. Insbesondere bevorzugt werden die erfindungsgemäßen Lösungen als keimfrei vorgesehen.

In allgemeiner Form betrifft daher die vorliegende Erfindung eine pharmazeutische Zusammensetzung in Form einer Lösung, umfassend Glukose und Octenidin, vorzugsweise wie in der Zusammensetzung Octenisept^{®}.

Der erfindungsgemäße Kit bzw. die Zusammensetzung wird insbesondere zur Herstellung eines Medikaments verwendet zur therapeutischen Verabreichung zur Behandlung von Wunden oder Verbrennungen oder zur Behandlung oder Vorbeugung von Infektionen, insbesondere Wundinfektionen. Vorzugsweise kommt die Zusammensetzung bzw. der Kit zur Behandlung von Wunden, Infektionen, insbesondere Wundinfektionen, Verbrennungen der Haut, einer Schleimhaut, insbesondere der Vagina, von inneren Organen, der Bauchhöhle, insbesondere bei chirurgischen Eingriffen zum Einsatz. Die erfindungsgemäßen Präparate erstellen somit ein besonders praktisches Mittel, welches effektiv bei chirurgischen Eingriffen als Desinfektionsmittel zum Einsatz kommen kann.

Die erfindungsgemäße pharmazeutische Zusammensetzung bzw. die Glukose- oder Octenidinlösung des Kits werden insbesondere bei physiologischem pH vorgesehen. Der pH-Wert kann somit beispielsweise zwischen 5 und 9 sein, vorzugsweise bis pH 8. Sofern dies notwendig ist, kann der pH-Wert durch geeignete Puffer, wie beispielsweise von organischen Säuren, wie Zitronensäure, Essigsäure, Fumarsäure oder Apfelsäure, sowie anorganischen Säuren, wie Salzsäure, Phosphorsäure oder Schwefelsäure, bzw. deren Alkali- bzw. Erdalkalimetallsalzen, insbesondere Natrium, Kalium, Magnesium und Calcium, bzw. durch Puffer von pharmazeutisch akzeptablen Basen, z.B. von NaOH, vorgesehen werden.

Insbesondere ist gemäß der vorliegenden Erfindung die Lösung von Octenidin bzw. der gesamten pharmazeutischen Zusammensetzung eine klare Lösung ohne präzipitierten Octenidin. Diese Lösung kann als solches in das Trägermaterial der vorliegenden Erfindung, wie beispielsweise ein Gel oder Hydrogel bzw. der Wundverband, vorgesehen werden.

Die vorliegende Erfindung wird anhand der beigefügten Zeichnungen und Beispiele noch näher erläutert ohne auf diese beschränkt zu sein. Darin zeigen:
Fig. 1: Inversmikroskopieaufnahme präzipitierter Octenidinkristalle aus einer Octenisept^{®}-NaCl-Lösung
Fig. 2: Modifikation der Octenisept^{®} induzierten Zellablösung durch Zusatz von NaCl oder Glukose im Zell-Detachment Assay mit Kristallviolett. a: graphische Balkendarstellung der Resultate: Balken repräsentieren den Mittelwert ± Standardabweichung von vier unabhängigen Experimenten. b: Fotographie der Zellrasen.
Fig. 3A und 3B: Modifikation der Octenisept^{®} induzierten Zellablösung durch Zusatz verschiedener Kohlenhydrate im Zell-Detachment Assay mit Kristallviolett.
Fig. 4: Konzentrationsabhängikeit von Glukose Zusätzen auf die Octenisept^{®} induzierte Zellablösung vom Kultursubstrat im Kristall-Violett Assay. Nach Waschen und Färben der adhärenten Zellen mit Kristallviolett wurde die Extinktion bei 550 nm gemessen. (Balken repräsentieren den Mittelwert ± Standardabweichung von 3 Messwerten). Die Verdünnung erfolgt ausgehend von einer 0,1% Octenidinlösung (Octenisept^{®}) mit einer 5% Glukoselösung in Anteilen von 0,1:200 bis 1:2.

### Beispiele:

Die ideale Konstellation zur Wundbehandlung ist I. die oberflächliche Zerstörung des mikrobiellen Biofilms auf der Wunde und II. die Stimulierung jener Prozesse der Wundheilung, die zu einer stabilen Abdeckung des darunter befindlichen Gewebes führen. Die Verankerung von proliferierenden Zellen mit dem Gewebsgerüst entspricht in der Zellkultur der Ausbildung von stabilen Verbindungen zwischen dem Zytoskelett, Integrinen und der Matrix am Boden der Kulturschalen. Um diesen Parameter zu quantifizieren wird die Resistenz gegen die Ablösung des Zellrasens bei mechanischer Beanspruchung wie Waschen bestimmt. Der verbleibende Zellrasen wird mit Kristallviolett gefärbt und mittels photometrischer Absorptionsmessung quantifiziert. Zusätzliche Effekte, die durch die Verwendung bestimmter Glukoselösungen in definierten Verdünnungen mit Octenisept^{®}, welche die Zelladhäsion verbessern und die beobachtete verbesserte Wundheilung erklären, werden gezeigt.

### Beispiel 1: Reagentien

Octenisept^{®} (Schülke&Mayr) Lösung (0,1% (w/w) Octenidindihydrochlorid und 2% (w/w) 2-Phenoxyethanol in H₂O); NaCl Lösung (0,9% (w/v) in H₂O); Glukose 5% (Mayrhofer Pharmazeutika) Glukose-Lösung 5% (w/v) in H₂O;

Laktose, 2-Deoxyglukose, Saccharose, Fructose, Polyethylenglykol, Chondroitin-4-sulphate, Chondroitin-6-sulphate, Dermatansulfat und Dextransulfat wurden in einer Endkonzentration von 5% (w/v) in H₂O sterilfiltriert verwendet.

Zur Inkubation mit Zellkulturen wurden gleiche Volumina von Octenisept^{®} und der jeweiligen Zusätze gemischt und bei 4°C bis zu einer Woche vor Verwendung gelagert.

### Beispiel 2: Zellkultur

Die humane Glioblastom-Zelllinie U373 wurde in Dulbecco's Modified Eagle Medium (DMEM) supplementiert mit 10% PCS (Gibco™), 2mM Glutamine (PAA) and 100 U/ml Penicillin/Streptomycin (PAA) bei 37°C und 5% CO₂ gezüchtet. Die Zellen wurden in Monolayer kultiviert und bei 80% Konfluenz passagiert. Vor der Durchführung der Zelladhäsionsexperimente wurden die Zellen in 96-well Platten transferiert und zur Konfluenz gebracht.

### Beispiel 3: Bestimmung der Zelladhäsion

Zelladhäsion wurde wie im Zitat im Detail beschrieben durchgeführt (Uchide, N. and H. Toyoda (2007) J Immunol Methods 328(1-2):215-9). Es wurden U373 Zellen in 96-well Platten mit Octenisept^{®} (Endkonzentration 5% (v/v) Octenisept^{®}, das entspricht 0,005% Octenidindihydrochlorid) singulär oder mit den angegebenen Zusätzen (5% v/v) 1 Stunde in glukosefreiem Leibovits Medium inkubiert. Nach Entfernen des Überstandes wurde die standardisierte Waschprozedur 3x angewendet und danach mit einer Motor-gesteuerten Pipette PBS zugegeben was zur Ablösung von nicht haftenden Zellen führte. Nur jene Zellen deren Matrix eine stabile Verbindung aufgebaut hatten wurden danach mit Glutaraldehyd fixiert und nach Entfernen des Glutaraldehyds mit Kristallviolett angefärbt. Der präzipitierte Farbstoff wurde mit einem SDS Puffer gelöst und die Extinktion bei 550 nm in einem ELISA Plattenleser gemessen.

### Beispiel 4: Präzipitations Assay

Die Stabilität der Löslichkeit der Komponenten von Octenisept^{®} in NaCl Lösung als ein Indikator für deren Interaktionen wurde durch Analyse der Präzipitate ermittelt. Nach Koinkubation von Octenisept^{®} mit dem gleichen Volumen von 0,9% w/v NaCl oder 5% Kohlenhydrat in sterilem Aqua-bidest für 3, 12, 24 und 72 Stunden bei 4°C wurden die Lösungen bei 2500 rpm, 5 min. zentrifugiert und das Präzipitat so vorhanden im Zeiss Inversmikroskop mit einer Digitalkamera mit dem METAVIEW Mikrophotographie-Dokumentationsystem ausgewertet.

### Beispiel 5: Zusatz von NaCl zu Octenisept^{®} führt zur Kristallbildung und Ausfällung:

Positiv geladenes Octenidin und negative Ladungen an der Bakterienoberfläche initiiert seine Wirksamkeit. Appliziert man wie bisher üblich Octenisept^{®} in einer 1:1 Verdünnung mit physiologischer Kochsalzlösung, so wird sich um das Octenidin Molekül eine Mischhydrathülle aus NaCl und dem Dipol Wasser aufbauen. Die hohe Ionenstärke bei Verdünnung mit NaCl_{physiol.} führt - ohne auf eine bstimmte Theorie beschränkt zu sein - zu einer Minderung der auf der positiven Ladung des Octenidin beruhenden bakteriziden Wirkung. Die Verdünnung mit NaCl beeinträchtigt somit die Wirksamkeit. Es wurden nun gezeigt, dass es sich dabei nicht nur um eine geringfügige Beeinträchtigung handelt, sondern insbesondere um eine induzierte Kristallbildung.

### Mikrophotografie des kristallinen Nieder Schlags:

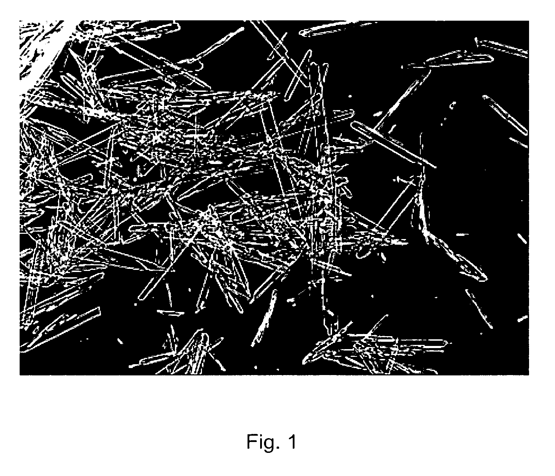

Nach 96 Stunden Inkubation bei 4°C entsteht bei Mischungen von Octenisept^{®} mit physiologischer Kochsalzlösung ein kristalliner Niederschlag (Fig. 1), der sich bei Erwärmen auf 60°C wieder löst (20 x Objektiv). In Mischungen von Octenisept^{®} mit 5% Glukose (1:1) ist auch nach 30 Tagen kein Niederschlag nachzuweisen. Andere polare, ungeladene Kohlenhydrate als Diluens bleiben ohne Kristallbildung, nicht aber sulfatierte polymere Kohlenwasserstoffe, wie Chondroitin-, Dermatan- oder Heparansulfate die zur raschen mikrokristallinen bzw. kolloidalen Trübung führen.

### Beispiel 6: Effekte von Glukose und NaCl Zusätzen auf die Octenisept^{®} induzierte Zellablösung vom Kultursubstrat im Kristall-Violett Assay

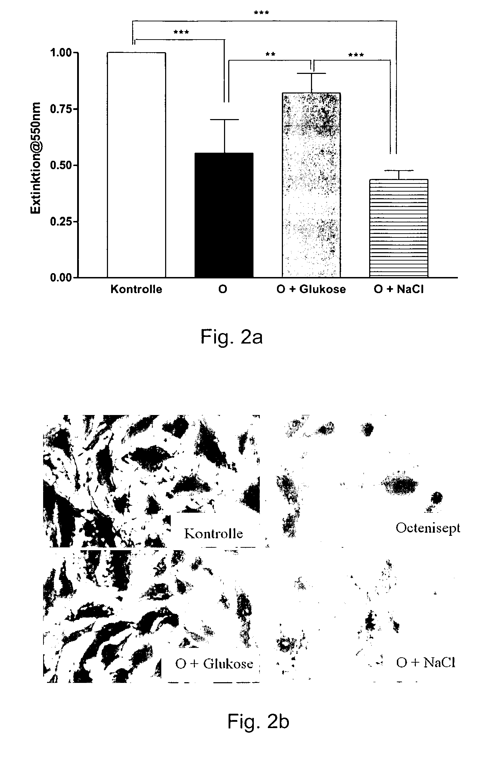

Der kristalline Niederschlag kann durch Verdünnung mit 5% Glukose-Lösung verhindert werden. Es wurde nun untersucht ob dieser Zusatz Effekte bewirkt, die zur Verbesserung der Wundheilung beitragen können. U373 Zellen wurden mit Octenisept^{®} alleine oder mit Zusätzen (1:1 mit Glukose 5% oder NaCl 0,9%) in Leibovits Medium für 1 Stunde inkubiert. Jeweils 5 µl der jeweiligen Lösungen wurden 100 µl Medium zugesetzt. Nach Waschen und Färben der adhärenten Zellen mit Kristallviolett wurde die Extinktion bei 550 nm gemessen (Fig. 2a). Die Extinktion der Kontrollen wurde auf 1 gesetzt. Statistische Signifikanzen wurden mittels Bonferroni's Multiple Comparison Test bestimmt (**: p<0,01, ***p<0,001).

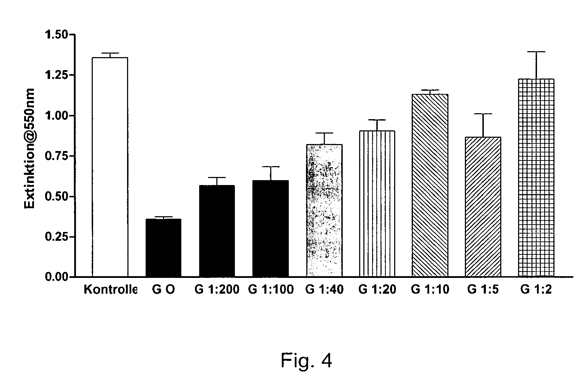

Zellen die mit Octenisept^{®} alleine (schwarzer Balken) behandelt wurden zeigten eine auf 50% gegenüber Kultivierung in Leibovits Medium reduzierte Stabilität die durch Verdünnung mit NaCl 0,9% noch geringer ausfiel (quergestreifter Balken). Mit Glukose 5% wurde die Resistenz gegen Ablösung hochsignifikant auf 80% des Kontrollwertes verbessert (grauer Balken). Eine Konzentrationsreihe mit Glukose ergab, dass der optimale Zusatz bei 2% in der Vorratslösung bzw. 0,1% Endkonzentration liegt (Fig. 4). Die Zahl der am Substrat haftenden Zellen nach Behandlung mit Octenisept^{®} +Glukose war signifikant größer als bei Octenisept^{®} +NaCl, wobei bei der höchsten Konzentration von Octenisept^{®} +NaCl sämtliche Zellen abgelöst waren während bei Octenisept^{®} +Glukose eine stabilere Verankerung bewirkte der einen intakten Zellrasen erhalten ließ (Fig.2b).

### Beispiel 7: Modifikation der Octenisept^{®} induzierten Zellablösung durch verschiedene Polyalkohole

Polyalkohole bilden eine breite Verbindungsklasse und es wurde daher untersucht, ob einzelne Untergruppen verschiedene Wirkung im Adhäsions-Test bewirkten. U373 Zellen wurden mit Octenisept^{®} alleine (-) oder mit Zusätzen (1:1 mit Polyalkohol 5%, Glukose, 2-Deoxyglukose (2-deoxyG.), Laktose, Saccharose, Fructose, Glucuronsäure, Polyethylenglykol (PEG), Chondroitin-4-sulfat (Ch.4-S.), Chondroitin-6-sulfat (Ch.6-S.), Dermatansulfat (Dermatan-S.) bzw. Dextransulfat (Dextran-S.)) in Leibovits Medium für 1 Stunde inkubiert mit jeweils 5 µl Zusatz zu 100 µl Medium. Nach Waschen und Färben der adhärenten Zellen mit Kristallviolett wurde die Extinktion bei 550 nm gemessen. (Balken repräsentieren den Mittelwert ± SD von 2 unabhängigen Experimenten (in Triplikaten gemessen). Die Extinktion der Kontrollen wurde auf 1 gesetzt. Statistische Signifikanzen wurden über den Bonferroni's Multiple Comparison Test bestimmt (**: p<0,01, ***p<0,001).

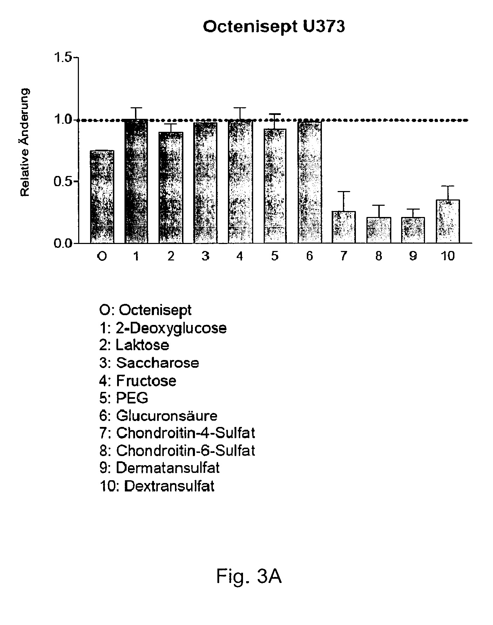

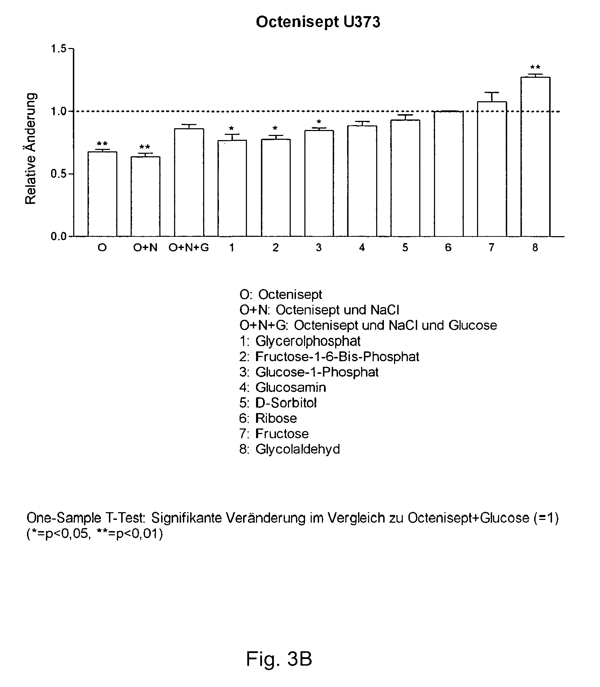

Aus Fig. 3 ist ersichtlich, dass wiederum Octenisept^{®} zu einer signifikanten Reduktion der Zelladhäsion führte, die durch polare Monomere wie Glukose, 2-Deoxyglukose, Fructose und Glucuronsäure, aber auch Disaccharide wie Laktose, Saccharose und Polymere wie Polyethylenglykol wieder verbessert wurde. Geladene Polymere wie Chondroitin-4-sulfat, Chondroitin-6-sulfat, Dermatansulfat und Dextransulfat konnte diese Wirkung nicht erzielen, reduzierten vielmehr signifikant die Adhäsion unter die Werte von NaCl.

Die Wirksamkeit von Polyalkoholen als Zusatz zu Octenidin wurde in Experimenten für ungeladene bzw. schwach saure Kohlenhydrate belegt. Die getesteten polymere sulfatierten Kohlenhydrate waren jedoch ohne Wirkung. Im Detail wirken Aldohexosen und Aldopentosen gleichwertig positiv und es ist kein Unterschied zwischen Aldosen und Ketosen in dieser Klasse vorhanden. Bei Derivaten ohne Aldehydgruppen ist eine geringfügig verminderte positive Wirkung zu verzeichnen (Zuckeralkohole, PEG). Glykosidische OH-Gruppen können wie am Beispiel Saccharose gezeigt gebunden sein ohne die Wirkung zu beeinträchtigen. Vielmehr scheinen saure Gruppen eine Rolle zu spielen die Kationen binden können. Glucuronsäure als innerer Ester ist demnach positiv wirksam nicht jedoch Phosphat- oder Sulfat derivatisierte Kohlenhydrate, die die Ablösung der Zellen durch Octenidin noch zusätzlich verstärken. Diese Befunde sind kongruent mit der antiseptischen Wirkungshypothese insofern, als eine durch Ladungsausgleich, wie sie durch starke Salzbildner zustandekommt, verminderte bakterizide Wirkung gefolgert wurde. Die Anwendung von Kohlenhydraten vermindert bei der Anwendung die physiologische Kochsalzkonzentration und unterstützt die bereits gut dokumentierte antiseptische Wirkung.

### Beispiel 8: Konzentrationsabhängikeit von Glukose Zusätzen auf die Octenisept^{®} induzierte Zellablösung vom Kultursubstrat im Kristall-Violett Assay

Um die optimale Konzentration bzw. Verdünnung zu ermitteln, bei der die Zubereitung mit Glukosezusatz die Zellablösung, die durch Octenisept^{®} alleine oder Octenisept^{®} verdünnt mit NaCl signifikant erniedrigt ist, wieder ausgleicht wurde eine Verdünnungsreihe von Zubereitungen mit Glukose 5% von 1:2 bis 1:200 verwendet. U373 Zellen wurden für eine Stunde mit Octenisept^{®} (1:20 im Medium) alleine (G0) bzw. mit Octenisept® und verschiedenen Volumina einer 5%igen Glukose-Lösung (resultierende Endverdünnungen 1:200 - 1:2 in Leibovits Medium) inkubiert. Die Effekte auf die Zelladhäsion wurden mittels Kristallviolett-Test bestimmt und in Fig.4 dargestellt.

Octenisept^{®} bewirkt eine erhebliche Reduktion der Zelladhärenz, die durch Zusatz von Glukose 1:200 oder 1:100 tendenziell verbessert wurde. Das Optimum des protektiven Effektes wurde bei einer Verdünnung von 1:20 ermittelt und zeigen bei weiter steigenden Konzentrationen starke Schwankungen aber keinen konstanten, wesentlich gesteigerten protektiven Effekt.

Im Experiment an Zellkulturen konnte bestätigt werden: Die Zahl der nach einem standardisierten Waschvorgang am Substrat haftenden Zellen nach Behandlung mit Octenisept^{®} +Glukose war signifikant größer als bei Octenisept^{®} + NaCl, wobei bei der höchsten Konzentration von Octenisept^{®} +NaCl sämtliche Zellen abgelöst waren während Octenisept^{®} +Glukose eine stabile Verankerung bewirkte. In Analogie zur Wundheilung in vivo indiziert dieser Parameter dass eine Octenisept^{®} + Glukose Verdünnung die Herstellung eines mechanisch stabilen Zellverbandes unterstützt und damit die Wundheilung beschleunigt (Cai et al. (2008) Invest Ophthalmol Vis Sei 49(5): 2163-71; Chiang et al. (1991) Dev Biol 146(2): 377-85; Guo et al. (2008) Cancer Investigation 26(4): 369 - 374; Wilkinson et al. (1994) Exp Dermatol 3(5): 239-45). Der optimale Einsatzbereich wurde bei einer 1:20 Verdünnung der 5% Glukoselösung bzw. 0,25% Glukose absolut in der Octeniseptlösung ermittelt.

## Patentansprüche

1. Octenidin oder ein pharmazeutisches Salz hiervon, insbesondere Octenidindihydrochlorid, bestimmt zur gemeinsamen therapeutischen Verabreichung des Octenidins in Lösung mit einem Polyalkohol der Formel 1: (H-C-OH)ₐ(HO-C-OH)_{b}(H-C-H)_{c},
wobei a, b, c ganze Zahlen sind, wobei a+b mindestens 2, vorzugsweise mindestens 3 ist, c ausgewählt ist aus 0, 1 oder einer Zahl aus dem Bereich von 2 bis a+b,
gegebenenfalls zusätzlich mit einer oder mehrerer Aldehydgruppen sofern diese (zyklische) Acetale mit einer der Hydroxygruppen bilden oder ein oder mehreren Ketogruppen gegebenenfalls als Acetal mit einer der Hydroxygruppen,
gegebenenfalls zusätzlich mit einer oder mehrerer Carbonsäuregruppen sofern der Polyalkohol ein zyklisches Acetal oder Acetal ist, vorzugsweise mit Ringgrößen von 5 bis 7 Atomen, bzw. eines Polymers, Polyethers oder Polyesters hiervon, mit der Maßgabe, dass sofern a+b 2 oder 3, ist der Polyalkohol als Polymer, Polyether oder Polyester vorliegt mit mindestens zwei Einheiten der Formel 1.

2. Kit umfassend ein Polyalkohol der Formel 1 wie in Anspruch 1 definiert und Octenidin oder pharmazeutische Salze hiervon, insbesondere Octenidindihydrochlorid, vorzugsweise bestimmt zur gemeinsamen therapeutischen Verabreichung in Lösung.

3. Pharmazeutisches Zusammensetzung in Form einer Lösung, umfassend einen Polyalkohol der Formel 1 wie in Anspruch 1 definiert und Octinidin oder pharmazeutische Salze hiervon, insbesondere Octenidindihydrochlorid.

4. Octenidin, Kit oder Zusammensetzung nach einem der Ansprüche 1 bis 3, **dadurch gekennzeichnet, dass** der Polyalkohol ein Kohlenhydrat der Summenformel 2: Cₙ(H2O)ₘ ist, wobei n eine ganze Zahl von mindestens 3 ist und m eine ganze Zahl im Bereich von n-15% bis n+15% (auf ganze Zahlen gerundet), vorzugsweise n-1, n oder n+1, ist.

5. Octenidin, Kit oder Zusammensetzung nach einem der Ansprüche 1 bis 4, **dadurch gekennzeichnet, dass** der Polyalkohol ungeladen ist.

6. Octenidin, Kit oder Zusammensetzung nach einem der Ansprüche 1 bis 5, **dadurch gekennzeichnet, dass** der Polyalkohol ein Mono- oder Disaccharid ist oder ein Deoxy- oder Monocarbonsäurederivat eines Mono- oder Disaccharids ist.

7. Octenidin, Kit oder Zusammensetzung nach einem der Ansprüche 1 bis 6, **dadurch gekennzeichnet, dass** der Polyalkohol eine Aldose oder Ketose umfasst, vorzugsweise eine Aldohexose, Aldopentose, Ketohexose oder eine Ketopentose.

8. Octenidin, Kit oder Zusammensetzung nach einem der Ansprüche 1 bis 7, **dadurch gekennzeichnet, dass** der Polyalkohol ausgewählt ist aus Glukose, Galaktose, Fruktose, Fukose, Maltose, Ribose, Deoxyribose, Deoxyglukose, insbesondere 2-Deoxyglukose, Saccharose, Laktose, Glucuronsäure, Polyethylenglykol, Sorbitol, insbesondere D-Sorbitol.

9. Octenidin, Kit oder Zusammensetzung nach einem der Ansprüche 1 bis 8, **dadurch gekennzeichnet, dass** der Polyalkohol und/oder Octenidin in Lösung ist, vorzugsweise in einer wässrige Lösung, vorzugsweise mit Wasser als reinem Lösungsmittel.

10. Octenidin, Kit oder Zusammensetzung nach einem der Ansprüche 1 bis 9, **dadurch gekennzeichnet, dass** der Polyalkohol in Lösung zur Verabreichung mit Octenidin eine Konzentration von 0,01% bis 12% (w/v), vorzugsweise von 0,1% bis 10%, insbesondere bevorzugst von 0,5% bis 7,5%, am meisten bevorzugt von 2,5% bis 5%, hat.

11. Octenidin, Kit oder Zusammensetzung nach einem der Ansprüche 1 bis 10, **dadurch gekennzeichnet, dass** Octenidin in Lösung zur Verabreichung mit dem Polyalkohol eine Octenidinkonzentration von 0,0001% bis 1% (w/w), vorzugsweise von 0,001% bis 0,1%, besonders bevorzugt von 0,002% bis 0,01%, hat.

12. Octenidin, Kit oder Zusammensetzung nach einem der Ansprüche 1 bis 11, **dadurch gekennzeichnet, dass** eine Lösung zur Verabreichung von Octenidin mit dem Polyalkohol eine NaCl-Konzentration unter 0,1% (w/v), vorzugsweise unter 0,05%, besonders bevorzugt unter 0,01%, am meisten bevorzugt unter 0,005%, hat.

13. Octenidin, Kit oder Zusammensetzung nach einem der Ansprüche 1 bis 12, **dadurch gekennzeichnet, dass** der Polyalkohol und/oder Octenidin in einem Träger vorliegt vorzugsweise ausgewählt aus einem Gel, vorzugsweise einem Hydrogel, oder einem Wundverband oder Tupfer, gegebenenfalls getränkt mit einer Lösung enthaltend den Polyalkohol und/oder Octenidin sowie langsam-freisetzende Träger.

14. Octenidin, Kit oder Zusammensetzung nach einem der Ansprüche 1 bis 13, **dadurch gekennzeichnet, dass** Octenidin und der Polyalkohol zur therapeutischen Verabreichung zur Behandlung von Wunden oder Verbrennungen, insbesondere der Haut, einer Schleimhaut, insbesondere der Vagina, oder der Bauchhöhle, insbesondere bei chirurgischen Eingriffen oder als Prophylaxe vor chirurgischen Eingriffen, oder zur Behandlung und Vorbeugung von Infektionen, insbesondere Wundinfektionen, vorzugsweise Infektionen von Verbrennungswunden, insbesondere der Haut, einer Schleimhaut, insbesondere der Vagina, der Bauchhöhle, von inneren Organen, insbesondere bei chirurgischen Eingriffen oder als Prophylaxe vor chirurgischen Eingriffen, bestimmt sind.

15. Pharmazeutische Mittel umfassend eine Lösung von Octenidin oder eines seiner pharmazeutisch akzeptablen Salze und Glukose mit einer Glukosekonzentration von 0,01% bis 12% (w/v), vorzugsweise von 0,1% bis 10%, insbesondere bevorzugt von 0,5% bis 7,5%, am meisten bevorzugt von 2,5% bis 5% und einer Octenidinkonzentration von 0,0001% bis 1% (w/w), vorzugsweise von 0,001% bis 0,1%, besonders bevorzugt von 0,002% bis 0,01%, gegebenenfalls zusammen mit einem Träger.

## Geänderte Patentansprüche

### Geänderte Patentansprüche gemäss Regel 137(2) EPÜ.

**1.** Octenidin oder ein pharmazeutisches Salz hiervon, insbesondere Octenidindihydrochlorid, bestimmt zur gemeinsamen therapeutischen Verabreichung des Octenidins in Lösung mit einem Polyalkohol der Formel 1: (H-C-OH)ₐ (HO-C-OH)_{b} (H-C-H)_{c},
wobei a, b, c ganze Zahlen sind, wobei a+b mindestens 2, vorzugsweise mindestens 3 ist, c ausgewählt ist aus 0, 1 oder einer Zahl aus dem Bereich von 2 bis a+b,
zusätzlich mit einer oder mehreren Aldehydgruppen sofern diese (zyklische) Acetale mit einer der Hydroxygruppen bilden oder ein oder mehreren Ketogruppen gegebenenfalls als Acetal mit einer der Hydroxygruppen,
gegebenenfalls zusätzlich mit einer oder mehreren Carbonsäuregruppen sofern der Polyalkohol ein zyklisches Acetal oder Acetal ist, vorzugsweise mit Ringgrößen von 5 bis 7 Atomen, bzw. einem Polymer, Polyether oder Polyester hiervon,
mit der Maßgabe, dass sofern a+b 2 oder 3 ist, der Polyalkohol als Polymer, Polyether oder Polyester vorliegt mit mindestens zwei Einheiten der Formel 1.

**2.** Kit umfassend ein Polyalkohol der Formel 1 wie in Anspruch 1 definiert und Octenidin oder pharmazeutische Salze hiervon, insbesondere Octenidindihydrochlorid, bestimmt zur gemeinsamen therapeutischen Verabreichung in Lösung.

**3.** Pharmazeutisches Zusammensetzung in Form einer Lösung, umfassend einen Polyalkohol der Formel 1 wie in Anspruch 1 definiert und Octinidin oder pharmazeutische Salze hiervon, insbesondere Octenidindihydrochlorid.

**4.** Octenidin, Kit oder Zusammensetzung nach einem der Ansprüche 1 bis 3, **dadurch gekennzeichnet, dass** der Polyalkohol ein Kohlenhydrat der Summenformel 2: Cₙ(H2O)ₘ ist, wobei n eine ganze Zahl von mindestens 3 ist und m eine ganze Zahl im Bereich von n-15% bis n+15% (auf ganze Zahlen gerundet), vorzugsweise n-1, n oder n+1, ist.

**5.** Octenidin, Kit oder Zusammensetzung nach einem der Ansprüche 1 bis 4, **dadurch gekennzeichnet, dass** der Polyalkohol ungeladen ist.

**6.** Octenidin, Kit oder Zusammensetzung nach einem der Ansprüche 1 bis 5, **dadurch gekennzeichnet, dass** der Polyalkohol ein Mono- oder Disaccharid ist oder ein Deoxy- oder Monocarbonsäurederivat eines Mono- oder Disaccharids ist.

**7.** Octenidin, Kit oder Zusammensetzung nach einem der Ansprüche 1 bis 6, **dadurch gekennzeichnet, dass** der Polyalkohol eine Aldose oder Ketose umfasst, vorzugsweise eine Aldohexose, Aldopentose, Ketohexose oder eine Ketopentose.

**8.** Octenidin, Kit oder Zusammensetzung nach einem der Ansprüche 1 bis 7, **dadurch gekennzeichnet, dass** der Polyalkohol ausgewählt ist aus Glukose, Galaktose, Fruktose, Fukose, Maltose, Ribose, Deoxyribose, Deoxyglukose, insbesondere 2-Deoxyglukose, Saccharose, Laktose, Glucuronsäure.

**9.** Octenidin, Kit oder Zusammensetzung nach einem der Ansprüche 1 bis 8, **dadurch gekennzeichnet, dass** der Polyalkohol und/oder Octenidin in Lösung ist, vorzugsweise in einer wässrige Lösung, vorzugsweise mit Wasser als reinem Lösungsmittel.

**10.** Octenidin, Kit oder Zusammensetzung nach einem der Ansprüche 1 bis 9, **dadurch gekennzeichnet, dass** der Polyalkohol in Lösung zur Verabreichung mit Octenidin eine Konzentration von 0,01% bis 12% (w/v), vorzugsweise von 0,1% bis 10%, insbesondere bevorzugst von 0,5% bis 7,5%, am meisten bevorzugt von 2,5% bis 5%, hat.

**11.** Octenidin, Kit oder Zusammensetzung nach einem der Ansprüche 1 bis 10, **dadurch gekennzeichnet, dass** Octenidin in Lösung zur Verabreichung mit dem Polyalkohol eine Octenidinkonzentration von 0,0001% bis 1% (w/w), vorzugsweise von 0,001% bis 0,1%, besonders bevorzugt von 0,002% bis 0,01%, hat.

**12.** Octenidin, Kit oder Zusammensetzung nach einem der Ansprüche 1 bis 11, **dadurch gekennzeichnet, dass** eine Lösung zur Verabreichung von Octenidin mit dem Polyalkohol eine NaCl-Konzentration unter 0,1% (w/v), vorzugsweise unter 0,05%, besonders bevorzugt unter 0,01%, am meisten bevorzugt unter 0,005%, hat.

**13.** Octenidin, Kit oder Zusammensetzung nach einem der Ansprüche 1 bis 12, **dadurch gekennzeichnet, dass** der Polyalkohol und/oder Octenidin in einem Träger vorliegt vorzugsweise ausgewählt aus einem Gel, vorzugsweise einem Hydrogel, oder einem Wundverband oder Tupfer, gegebenenfalls getränkt mit einer Lösung enthaltend den Polyalkohol und/oder Octenidin, sowie langsam-freisetzende Träger.

**14.** Octenidin, Kit oder Zusammensetzung nach einem der Ansprüche 1 bis 13, **dadurch gekennzeichnet, dass** Octenidin und der Polyalkohol zur therapeutischen Verabreichung zur Behandlung von Wunden oder Verbrennungen, insbesondere der Haut, einer Schleimhaut, insbesondere der Vagina, oder der Bauchhöhle, insbesondere bei chirurgischen Eingriffen oder als Prophylaxe vor chirurgischen Eingriffen, oder zur Behandlung und Vorbeugung von Infektionen, insbesondere Wundinfektionen, vorzugsweise Infektionen von Verbrennungswunden, insbesondere der Haut, einer Schleimhaut, insbesondere der Vagina, der Bauchhöhle, von inneren Organen, insbesondere bei chirurgischen Eingriffen oder als Prophylaxe vor chirurgischen Eingriffen, bestimmt sind.

**15.** Pharmazeutische Mittel umfassend eine Lösung von Octenidin oder eines seiner pharmazeutisch akzeptablen Salze und Glukose mit einer Glukosekonzentration von 0,01% bis 12% (w/v), vorzugsweise von 0,1% bis 10%, insbesondere bevorzugt von 0,5% bis 7,5%, am meisten bevorzugt von 2,5% bis 5% und einer Octenidinkonzentration von 0,0001% bis 1% (w/w), vorzugsweise von 0,001% bis 0,1%, besonders bevorzugt von 0,002% bis 0,01%, gegebenenfalls zusammen mit einem Träger.
